# EUROPEAN PATENT SPECIFICATION

(11) **EP 2 259 753 B1**
(45) Date of publication and mention of the grant of the patent: **12.11.2014**
(21) Application number: 09717686.1
(22) Date of filing: 05.03.2009
(51) Int. Cl.: A61F 2/38, A61F 2/30

(54) **METHOD OF MAKING AN EDGE-MATCHED ARTICULAR IMPLANT**
VERFAHREN ZUR HERSTELLUNG EINES GELENKIMPLANTATS MIT ANGEPASSTEM RAND
PROCÉDÉ DE FABRICATION D'UN IMPLANT ARTICULAIRE À BORDS CONCORDANTS

(30) Priority: 05.03.2008 US 34014
(43) Date of publication of application: 15.12.2010
(73) Proprietor: ConforMIS, Inc., Burlington, MA 01803 (US)
(72) Inventor: LANG, Philipp, Lexington MA 02421 (US); STEINES, Daniel, Lexington MA 02421 (US); FITZ, Wolfgang, Sherborn MA 01770 (US)
(74) Representative: Grund, Martin
(86) International application number: PCT/US2009/036218
(87) International publication number: WO 2009/111656

(56) References cited:
- EP-A2- 1 754 457
- WO-A1-2007/090784
- US-A1- 2007 233 269
- US-A1- 2007 233 269
- US-B1- 6 500 208

## Description

### Technical Field

The embodiments described herein relate to a method of making a tibial plateau implant for repairing a knee joint of a patient. The closest prior art is document WO 2007/090784 A1, which defines a tibial plateau implant configured to be fit into a tibial plateau of a patient, the implant comprising a substantially tray-shaped body having an inferior surface, a superior surface, and an outer peripheral edge extending between the inferior and superior surface; wherein at least a portion of the peripheral edge is configured to substantially match at least a corresponding portion of the periphery of the tibial plateau when the tibial plateau implant is implanted on the tibial plateau.

### Background Art

It is common to resect the upper tibia during, for example, unicompartmental knee arthroplasty (and also bicompartmental and total knee arthroplasty), and insert a tibial implant. Typically, the tibial implant is selected from a limited, fixed number of sizes such that overhang of the implant over the resected tibial plateau is prevented. Towards this end, the tibial implant is often selected such that the implant's periphery is smaller than the tibial plateau's outer periphery. Alternatively, in select cases, the implant may overhang with the potential for interference with adjacent soft-tissues and ligaments. As a result, the implant rests on the inner cancellous bone rather than the harder, outer cortical bone located at the outer periphery of the tibial plateau. As cancellous bone is spongy, the tibial implant will tend to shift position after time.

### Summary

One example is an implant configured to be fit into a cut portion of bone of a patient. The implant can include a substantially flat or substantially tray-shaped or dome-shaped body having an inferior surface, a superior surface, and an outer peripheral edge extending between the inferior and superior surface. At least a portion of the peripheral edge can be configured to substantially match at least a corresponding portion of the periphery of the cut portion of the bone such that the implant is substantially supported by cortical bone.

Another example is a tibial tray configured to be fit into a tibial plateau of a patient. The tray can include a substantially flat or substantially tray-shaped or dome-shaped body having an inferior surface, a superior surface, and an outer peripheral edge extending between the inferior and superior surface. At least a portion of the peripheral edge can be configured to substantially match at least a corresponding portion of the periphery of the tibial plateau when the tibial tray is implanted on the tibial plateau.

This example can include an anchor for securing the tibial tray. An entire outer portion of the peripheral edge can be configured to substantially match the corresponding periphery of the tibial plateau when the tibial tray is implanted on the tibial plateau, and an entire inner portion of the peripheral edge substantially abuts, optionally, a vertical cut in the tibial plateau. Alternatively, at least a portion of the peripheral edge can be configured to be recessed from a corresponding periphery of the tibial plateau and can be further configured to be adjacent to cortical bone when the tibial tray is implanted on the tibial plateau. In still another embodiment, at least a first outer portion of the peripheral edge can be configured to be adjacent to cortical bone and at least a second outer portion of the peripheral edge can be configured to be adjacent to cancellous bone when the tibial tray is implanted on the tibial plateau.

Additionally, the superior surface of the tray can be at least partially derived from patient-specific data of a femoral condyle. The patient-specific data can be obtained from an image of a femoral condyle, an image of a tibia or tibial plateau or a shape of an implant. The superior surface can comprise at least one curve derived from patient-specific data. The superior surface can be made of Ultra High Weight Molecular Polyethylene (UHMWPE) as well as cross-linked polyethylene.

The contour of the peripheral edge can be derived from patient-specific data, which can be derived, for example, from an image of a proximal end of a tibia. The peripheral edge of the tibial tray can be configured to rest on cortical bone when implanted on the tibial plateau. The tray can have a thickness from the inferior surface to the superior surface of about 3 to 15 mm.

In accordance the invention, a method of making a tibial plateau implant for repairing a knee joint of a patient is defined in claim 1.

The periphery of the implant may include an inner edge which spans across the tibial plateau. Deriving the outer periphery of the tibial plateau may include deriving a three-dimensional representation. The image of the tibial plateau may include subchondral bone, cortical bone, normal articular cartilage and/or diseased articular cartilage. The implant may further include an anchor for securing the implant. The anchor may be a keel, peg, nub, and/or rod. The implant may include a polymer(s), a ceramic(s), a metal(s) and/or a ceramic-metal composite(s). The implant may further include an inferior surface for facing the tibial plateau, and a superior surface for facing the femur, and wherein the superior surface includes at least one of a ceramic, a metal, a polymer and a ceramic-metal composite. The implant may further include an inferior surface for facing the tibial plateau, and a superior surface for facing the femur, the method further including deriving, at least partially, the superior surface from patient-specific data of a femoral condyle. The patient specific data may be obtained from an image of a femoral condyle. The outer edge may be adapted to substantially rest on cortical bone on the tibial plateau. The method may further include securing the implant to the tibial plateau, wherein the outer edge of the implant rests substantially on cortical bone. The implant may have a thickness of about 3 to 15 mm. At least a section of the tibial plateau may be resected.

In other embodiments, methods of making a tibial plateau implant can include obtaining an image of a knee joint including a tibial plateau. A representation of the outer tibial edge of the tibial plateau is derived from the image. An implant body is provided having a bearing surface, a tibial interface, an inner edge, and an outer edge that substantially matches the contour of the outer tibial edge.

In related embodiments, the outer edge may be adapted to substantially rest on cortical bone on the tibial plateau. The implant may be secured to the tibial plateau, wherein the outer edge of the implant rests substantially on cortical bone.

Methods of joint arthroplasty may include providing an implant for a tibial plateau having a body with a bearing surface, a tibial interface, an inner edge, and an outer edge that substantially matches the contour of the outer edge of a patient's tibial plateau. The tibial plateau can be prepared to receive the implant. The implant can be secured to the prepared implant site, wherein at least portions of the outer periphery of the tibial interface rests substantially on cortical bone.

The implant may include an anchor. The anchor may be a keel, peg, nub, and/or rod. The implant may include a bearing surface component for receiving a femoral condyle. The bearing surface component may include ceramic(s), metal(s), polymer(s) and/or ceramic-metal composite(s). The polymer may include UHMWPE. The bearing surface may be at least partially derived from patient-specific data of a tibial plateau including subchondral bone, cortical bone, normal and/or diseased cartilage, one or more femoral condyles including subchondral bone, cortical bone, normal and/or diseased cartilage. The patient-specific data may be obtained from an image of a femoral condyle or a tibial plateau or a first or an opposing second articular surface. The body may include polymer(s), ceramic(s), metal(s) and/or ceramic-metal composite(s). The contour of the outer edge may be derived from patient-specific data. The patient-specific data may be obtained from an image of a proximal tibial end. The implant may have a thickness of about 3 to 15 mm. The method may further include obtaining an image of at least a portion of the tibial plateau, and deriving the outer edge of the implant based, at least in part, on the image. The inner edge of the implant may be adapted to span across the tibial plateau. The undersurface of the implant may be flat or curved. The undersurface and/or the top surface of the implant may be at an angle other than 90 degrees relative to the sagittal or coronal axis of the tibia or the biomechanical axis.

The implant may include a body having a bearing surface, a tibial interface, an inner edge, and an outer edge that substantially matches the contour of the outer edge of a patient's tibial plateau.

Any implant, e.g. in a knee, hip, shoulder or other joint, may be made of a single material, e.g. polyethylene. The implant may also be made using two materials, e.g. a metal backing and a polyethylene insert. The polyethylene insert may be locked inside the metal backing using standard locking mechanisms as are known in the art.

The implant may include an anchor for securing the implant. The anchor may be a keel, peg, nub, and/or rod. Portions of the implants, e.g. the pegs or keel or portions of the bone facing surface may be porous coated. The undersurface of the implant may include cement pockets. The cement pockets may be open at the external margin of the implant to interface with the endosteal bone of the cut tibial plateau or the cut surface along a potential vertical cut, when used. The implant may further include a bearing surface component for receiving a femoral condyle. The bearing surface component may include a ceramic(s), metal(s), polymer(s) and/or ceramic-metal composite(s). The polymer may include UHMWPE. The bearing surface may be at least partially derived from patient-specific data of a femoral condyle or a tibial plateau or it may reflect the shape or be a mirror image of aspects of the external geometry of the femoral bearing surface, e.g. in different flexion or extension angles. The patient-specific data may be obtained from an image of a femoral condyle or a tibial plateau. The body may include polymer(s), ceramic(s), metal(s) and/or ceramic-metal composite(s). The contour of the outer edge may be derived from patient-specific data. The patient-specific data may be obtained from an image of a proximal tibial end. The outer periphery of the tibial interface may be adapted to substantially rest on cortical bone. The implant may have a thickness of about 3 to 15 mm.

The contour of the outer edge of the implant may be derived from patient-specific data. The patient-specific data may be obtained from an image of a proximal tibial end. The contour of the outer edge of the implant may be derived from an axial or near-axial cross-sectional image, a sagittal or near-sagittal cross-sectional image, a coronal or near coronal cross-sectional image, or any other cross-sectional image of the proximal tibia. Alternatively, the contour of the outer edge of the implant may be determined by creating a virtual model of the proximal tibia and performing a virtual cut on the model. The virtual cut performed on the virtual model may take into account one or more mechanical or anatomical axes of the knee.

Since the implant will be edge matched, the external contour of the implant can be convex, but it can also include concave portions. For example, in a hip joint, concave shapes can be integrated in the external contour of the device in order to achieve near 100% congruency with the shape of the acetabular rim.

### Brief Description of the Drawings

The foregoing features will be more readily understood by reference to the following detailed description, taken with reference to the accompanying drawings, in which:
**FIG. 1** is a side perspective view of a resected tibial plateau;
**FIG. 2** is a side perspective view of a tibial implant asserted on the resected tibial plateau of **FIG. 1****;**
**FIG. 3** is a diagram of a method of joint arthroplasty;
**FIG. 4** is a side perspective view of a virtual model of a proximal end of a tibia with a virtual tibial cut;
**FIG. 5** is a side perspective view of an alternate embodiment of a tibial implant having two components asserted on two resected tibial plateaus respectively;
**FIG. 6** is a side perspective view of an alternate embodiment of a tibial implant asserted on the resected tibial plateau of **FIG. 1****;**
**FIG. 7** is a side perspective view of an alternate embodiment of a tibial implant asserted on the resected tibial plateau of **FIG. 1****;**
**FIG. 8** is a side perspective view of an alternate embodiment of a tibial implant asserted on a resected tibial plateau.
**FIG.** 9 is a side perspective view of an alternate embodiment of a tibial implant asserted on a resected tibial plateau extending across the entire proximal end of the tibia.

### Detailed Description

Various methods, systems and devices for joint arthroplasty are described to provide an implant for a tibial plateau that has an outer edge that substantially matches or corresponds to the outer periphery of the tibial plateau, either the entire periphery or at least a portion of the periphery. Preferably, the outer edge of the implant rests entirely on cortical bone to provide support for the implant. However, in some embodiments, only a portion of the outer edge of the device will rest on cortical bone.

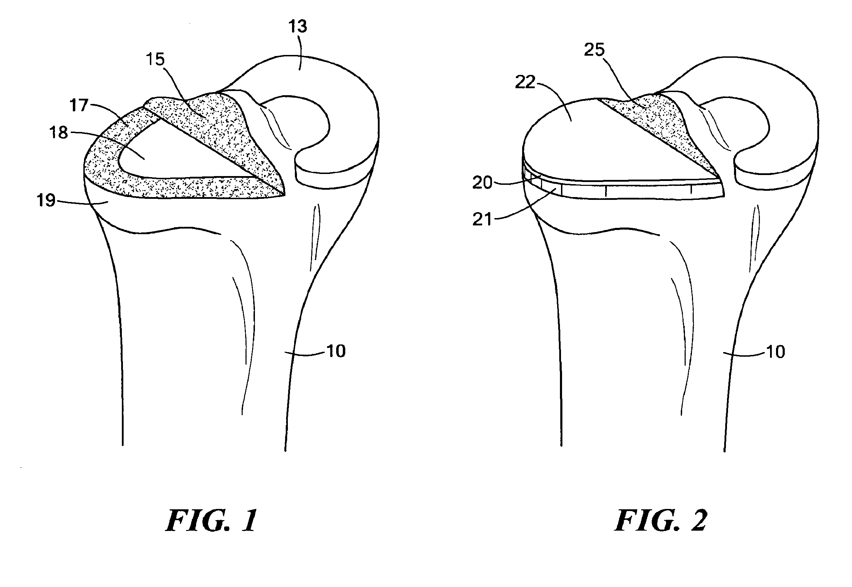

Referring to **FIG. 1****,** a resected portion of an upper (proximal) end of a tibia **10** is illustrated. The medial tibial plateau of the left tibia **10** is resected, and a lateral compartment of the tibia **10,** including a meniscus **13,** is left intact. In alternate embodiments, the lateral portion of the tibia **10** may be resected, instead, or both lateral and medial portions of the tibia **10** may be resected. Furthermore, the implant is not limited to the tibia, and may be applied to other joint surfaces where it is advantageous to have the implant rest on the outer periphery of the bone.

The resected lateral compartment of the tibia **10** may be cut, for example, along a sagittal plane to create a side wall **15.** This cut, in combination with a horizontal cut, forms a generally flat, resected tibial surface onto which a tibial implant **20** may be placed, as shown in **FIG. 2****,** described below.

As shown in **FIG. 1**, the resected tibial surface includes an outer periphery **19** made of cortical bone **17.** Within the outer periphery of the resected tibial surface lies cancellous bone **18,** which is spongy compared to the hard cortical bone **17.**

Referring to **FIG. 2**, an implant **20** is a tibial implant for use with a corresponding articulating femoral resurfacing or replacement implant. Implant **20** has an outer peripheral edge **21** that substantially matches the outer periphery of the tibial plateau. The matched edge ensures that the outer portion of implant **20** rests on cortical bone **17**, which provides better support than cancellous bone **18.** The outer edge **21** of the implant **20** is fully supported by the hard cortical bone **17,** as opposed to resting on the spongy cancellous bone **18.** It is to be understood that an inner peripheral edge **25** of implant **20** may span across, and may or may not contact, the tibial plateau, including cancellous bone.

The implant **20** includes an inferior surface (not shown in **FIG. 2**) that faces the tibial plateau, and a superior surface **22** that faces the femur. At least a portion of the superior surface **22** is load bearing. The superior surface **22** may be made of, for example, a ceramic, a metal, a polymer and/or a ceramic-metal composite. The inferior and superior surfaces may be derived, at least partially, from patient specific data of a femoral condyle, and/or may be matched to or defined by a curve of a corresponding femoral implant component. The patient specific data may be obtained from an image of a femoral condyle. The thickness of the implant between the superior and inferior surfaces may be about 3 to 15mm. Other structures are possible. For example, the sidewalls of a tibial tray can be rounded, tapered, recessed, proud or flush relative to the peripheral edge of the tibial plateau.

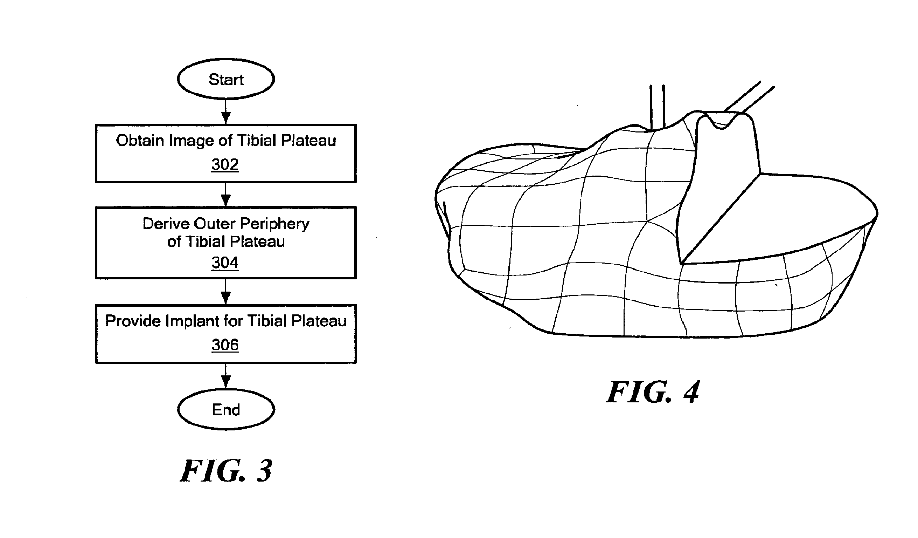

**FIG. 3** shows a method of joint arthroplasty. The method begins at step **302,** in which an image(s) of at least a portion of the tibial plateau is obtained. The obtained image may be a result of, without limitation, an MRI, CT, spiral CT, x-ray, ultrasound, digital tomosynthesis, and/or optical coherence tomography. The image of the tibial plateau may include subchondral bone, cortical bone, normal articular cartilage and/or diseased articular cartilage.

The method continues to step **304,** in which an outer periphery of at least a portion of the tibial plateau is derived, based on the image. This may be performed electronically. For example, the derivation may be performed, without limitation, by a processor (e.g., a microprocessor, microcontroller, digital signal processor, or general purpose computer), programmable logic for use with a programmable logic device (e.g., a Field Programmable Gate Array (FPGA) or other PLD), discrete components, integrated circuitry (e.g., an Application Specific Integrated Circuit (ASIC)), memory, or any other means including any combination thereof. Memory may include, for example, a diskette, a fixed disk, a Compact Disk (CD), Read Only Memory (ROM), Erasable Programmable Read-Only Memory (EPROM), and/or Random Access Memory (RAM). Computer program logic implementing all or part of the functionality previously described herein may be embodied in various forms, including, but in no way limited to, a source code form, a computer executable form, and various intermediate forms (e.g., forms generated by an assembler, compiler, linker, or locator.) Source code may include a series of computer program instructions implemented in any of various programming languages (e.g., an object code, an assembly language, or a high-level language such as Fortran, C, C++, C#, JAVA, or a scripting language) for use with various operating systems or operating environments. The source code may define and use various data structures and communication messages. The source code may be in a computer executable form (e.g., via an interpreter), or the source code may be converted (e.g., via a translator, assembler, or compiler) into a computer executable form.

Deriving the outer periphery may include deriving a cross-sectional (for example, an axial or near axial, a sagittal or near sagittal, coronal or near coronal cross-section), a two-dimensional, or a three-dimensional representation of the proximal tibia. Various scan planes may be combined to form the three dimensional representation. It may also include simulating the tibial cut on a series of two dimensional displays or on a three dimensional representation, as shown, for example, in **FIG. 4****.** The direction of the simulated tibial cut may be based on one or more mechanical or anatomical axes of the knee. These one or more axes can be derived from the same image or from one or more separate images registered into the same coordinate system as the tibial image. The height of the simulated cut may be determined from one or more reference points or landmarks on the tibia or the femur in the image. As the tibia has a taper, the outer periphery of the tibial plateau gets smaller moving in the superior to inferior direction. In preferred embodiments, the virtually derived outer periphery of the tibial plateau is thus determined at a desired cut height.

The method then continues to step **306,** in which an implant is provided for the tibial plateau. The implant has a periphery that includes an outer edge that substantially matches the derived outer periphery of the tibial plateau. In preferred embodiments, the outer edge of the tibial implant thus advantageously rests on cortical bone, as described above. The implant may be made of, without limitation, a polymer, a ceramic, a metal, and/or a ceramic metal composite.

The method may then include securing the implant to the tibial plateau. The tibial plateau may be resected, as shown for example, in **FIG. 1****.** The implant provided may include an anchor for securing the implant to the tibial plateau. The anchor may be, without limitation, a keel, peg, nub and/or rod.

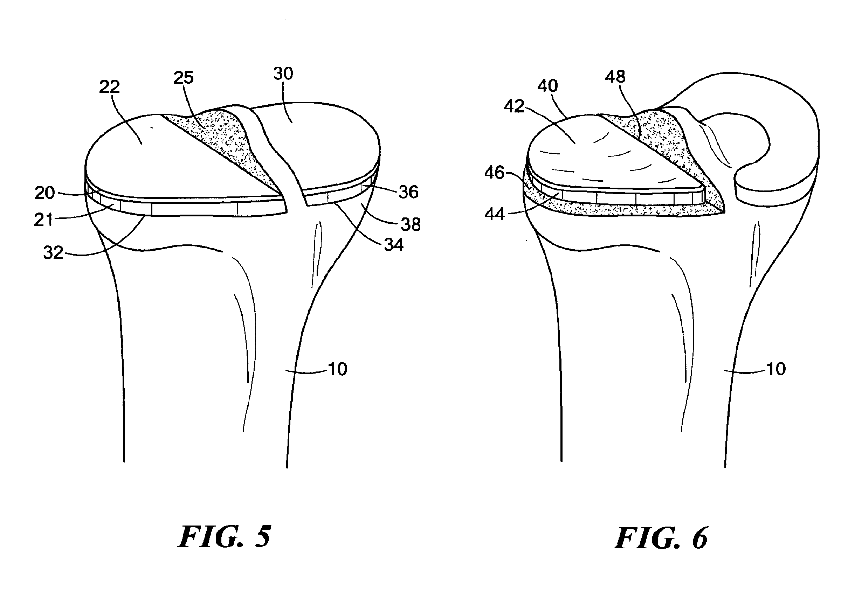

Many other variations are possible. Referring to **FIG. 5****,** one alternate example is an implant having two separate tibial components **20** and **30.** When implanted, each component rests on a separate tibial plateau **32** and **34.** Tibial components **20** and **30** are structurally similar and made from the same materials (but could be different structures and/or materials in other examples). However, each is sized to correspond to match the outer periphery of the tibial plateaus **32** and **34.** Thus, tibial component **30** includes an outer sidewall **36** that substantially corresponds to an outer periphery **38** of tibial plateau **34.** Tibial components **20** and **30** could be used, for example, with a bi-compartmental resurfacing device (such as the ConforMIS iDuo), a total knee resurfacing device (such as the ConforMIS iTotal), or a total knee replacement device.

In another example, as shown in **FIG. 6**, tibial tray **40** is similar in shape, structure and materials to implant **20.** A superior surface **42** of tibial tray **40** is made of UHWMPE and has a concavity in the coronal plane designed to match the curve of a corresponding femoral implant component. The curve preferably is 5 times the radius of the curve of the femoral implant in the coronal plane, but many other examples are possible. The outer edge **44** of tibial tray **40** does not extend completely to the outer edge **46** of the tibial plateau, but the outer edge **44** does rest on cortical bone. In still other examples, a portion of the margin of the tibial implant can be flush with the periphery of the bone while other portions are recessed from the periphery yet still lie on cortical bone. In other examples, one or more portions of the margin of the tibial implant can be flush with the periphery of the bone, one or more other portions can be recessed from the periphery yet still lie on cortical bone, and one or more other portions can be recessed from the periphery and lie over cancellous bone. In the case of the portion that lies on cancellous bone, some or all of the periphery can contact the bone or be raised up from the bone.

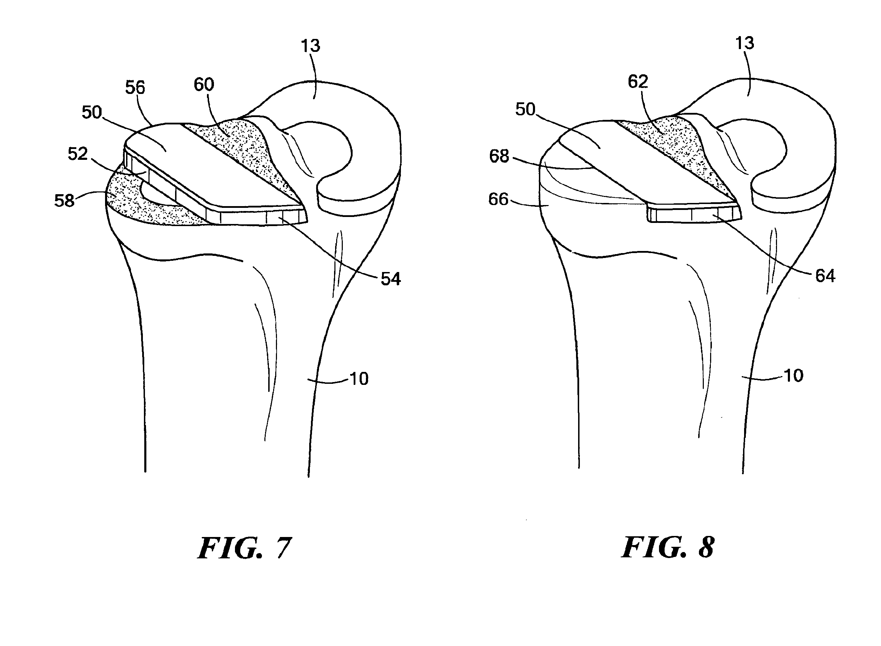

Referring to **FIG. 7**, another example includes a narrower tibial tray **50** that has a periphery that is matched to the periphery **58** of the patient's cortical bone only at end portions **54** and **56,** while side portion **52** extends across cancellous and cortical bone. The far side of the implant **50** abuts the vertical tibial cut **60.**

Referring to **FIG. 8****,** another example includes tibial tray **50,** but tibial tray **50** is inset into an alternative tibial plateau **62** that includes a recess portion **64** and an uncut portion **66.** In this example, an upper peripheral edge **68** is matched to the uncut subchondral bone, or, alternatively, uncut cartilage.

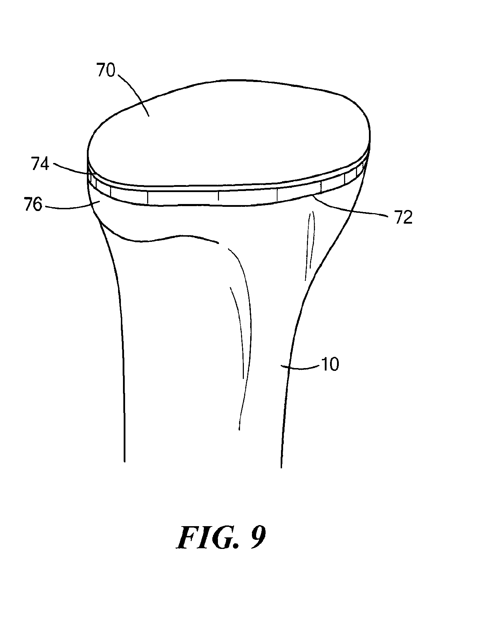

Referring to **FIG. 9****,** another example includes tibial tray **70** that rests on tibial plateau **72.** Tibial plateau **72** is cut and extends across the entire portion of the proximal end of tibia **10.** Tibial tray **70** has a peripheral side **74** that is matched to the periphery **76** of the patient's cortical bone.

Another example is an implant for a shoulder joint. In a shoulder joint, the glenoid rim can substantially support the implant. The implant can be shaped or selected using an imaging test such as a CT scan or MRI scan to substantially fit onto the glenoid rim.

In another example, in a hip joint, the acetabular rim can substantially support the implant. The implant can be shaped or selected using an imaging test such as a CT scan or MRI scan to substantially fit onto the acetbular rim. The implant can be secured to the acetabular rim, for example, by forming a lip around the outer edge of the implant that rest directly on the acetabular rim or on a bone cut around the acetabular rim that exposes a flat bone surface that engages the lip of the implant when inserted. Such examples can provide improved structural support for implants in these joints. Such improved structural support may improve the wear and lifetime of the implants. For example, a major cause of implant failure in hip joints is loosening of the implant. Providing a improved structural support, which is advantageous

Other examples can apply to orthopedic implants for other joints and bones where a portion of the implant is sized to correspond to the periphery of an uncut or cut portion of bone of a patient. In such implants, the cortical bone can support most or all of the load placed on the implant at the bone-implant interface. At least a portion of the peripheral edge can be configured to substantially match at least a corresponding portion of the periphery of a cut or uncut portion of the bone such that the implant is substantially supported by cortical bone or by the rim of the articular structure. Many other examples are possible.

The embodiments described above are intended to be merely exemplary; many other embodiments including various combinations of the elements described above or other additional elements and/or additional embodiments are possible. All such variations and modifications are intended to be within the scope of the invention as defined by the appended claims.

## Claims

1. A method of making a tibial plateau implant for repairing a knee joint of a patient, the method comprising:
obtaining electronic image data of the knee joint including at least a portion of the tibial plateau of the knee joint;
deriving an outer periphery of at least a portion of the tibial plateau based on the image data, said deriving comprising simulating a cut using the electronic image data, wherein the cut is in a predetermined orientation relative to one or more mechanical or anatomical axes of the knee; and
designing or selecting an implant (20) having a periphery that includes an outer edge (21), at least a portion of which is configured to substantially match at least a portion of the derived outer periphery of the tibial plateau and to rest on cortical bone of the tibia.

2. The method of claim 1, wherein using the electronic image data includes creating a two dimensional representation, a three dimensional representation, or both of the knee joint including a portion of the tibia.

3. The method of claim 1, wherein the one or more axes of the knee joint are determined using the electronic image data and/or additional image data.

4. The method of claim 1, wherein the one or more axes of the knee is a mechanical axis.

5. The method of claim 1, wherein the one of more axes of the knee is an anatomical axis.

6. The method of claim 1, wherein simulating the cut includes determining a height of the cut based on a reference point or a landmark of the knee joint.

7. The method of claim 1, including configuring at least a portion of the outer periphery of the implant to rest on a cancellous bone legion of the simulated cut surface of the tibia.

8. The method of claim 1, including configuring at least a portion of the outer edge of the periphery of the implant to be flush with the corresponding portion of the derived outer periphery of the simulated cut surface of the tibia.

9. The method of claim 1, including configuring at least a portion of the outer edge of the. periphery of the implant to recess from the corresponding portion of the derived outer periphery of the simulated cut surface of the tibia.

## Patentansprüche

1. Ein Verfahren zur Herstellung eines Tibiaplateau-Implantats zur Reparatur eines Kniegelenks eines Patienten, das Verfahren umfassend:
Erhalten elektronischer Bilddaten des Kniegelenkes umfassend mindestens einen Teil des Tibiaplateaus des Kniegelenks;
Ableiten einer äußeren Peripherie von mindestens einem Teil des Tibiaplateaus auf Grundlage der Bilddaten, wobei jenes Ableiten Simulieren eines Schnitts mit Hilfe der elektronischen Bilddaten umfasst, wobei der Schnitt in einer vorgegebenen Orientierung relativ zu ein oder mehr mechanischen oder anatomischen Achsen des Knies ist; und
Entwerfen oder Auswählen eines Implantats (20) mit einer Peripherie, die einen äußeren Rand (21) umfasst, wobei mindestens ein Teil davon konfiguriert ist, um mindestens einem Teil der abgeleiteten äußeren Peripherie des Tibiaplateaus im Wesentlichen zu entsprechen und um auf kortikalem Knochen der Tibia aufzuliegen.

2. Das Verfahren nach Anspruch 1, wobei unter Verwendung der elektronischen Bilddaten Erzeugen einer zweidimensionalen Darstellung, einer dreidimensionalen Darstellung, oder beidem, des Kniegelenks, einschließlich eines Teils der Tibia, umfasst.

3. Das Verfahren nach Anspruch 1, wobei die ein oder mehr Achsen des Kniegelenks unter Verwendung der elektronischen Bilddaten und/oder zusätzlichen Bilddaten bestimmt werden.

4. Das Verfahren nach Anspruch 1, wobei die ein oder mehr Achsen des Kniegelenks eine mechanische Achse ist.

5. Das Verfahren nach Anspruch 1, wobei die ein oder mehr Achsen des Kniegelenks eine anatomische Achse ist.

6. Das Verfahren nach Anspruch 1, wobei die Simulation des Schnitts das Bestimmen einer Höhe des Schnitts auf Grundlage eines Referenzpunkts oder Orientierungspunkts des Kniegelenks umfasst.

7. Das Verfahren nach Anspruch 1, umfassend Konfigurieren mindestens eines Teils der äußeren Peripherie des Implantats, um auf einem spongiösen Knochenbereich der simulierten Schnittfläche der Tibia aufzuliegen.

8. Das Verfahren nach Anspruch 1, umfassend Konfigurieren mindestens eines Teils des äußeren Randes der Peripherie des Implantats, um mit dem entsprechenden Teil der abgeleiteten äußeren Peripherie der simulierten Schnittfläche der Tibia bündig zu sein.

9. Das Verfahren nach Anspruch 1, umfassend Konfigurieren mindestens eines Teils des äußeren Randes der Peripherie des Implantats, um den entsprechenden Teil der abgeleiteten äußeren Peripherie der simulierten Schnittfläche der Tibia auszusparen.

## Revendications

1. Procédé de fabrication d'un implant de plateau tibial pour réparer une articulation de genou d'un patient, le procédé comprenant :
l'obtention de données d'image électronique de l'articulation de genou comprenant au moins une partie du plateau tibial de l'articulation de genou ;
la déduction d'une périphérie extérieure d'au moins une partie du plateau tibial sur la base des données d'image, ladite déduction comprenant la simulation d'une découpe en utilisant les données d'image électronique, dans lequel la découpe est dans une orientation prédéterminée relativement à un ou plusieurs axes mécaniques ou anatomiques du genou ; et
la conception ou la sélection d'un implant (20) ayant une périphérie qui comprend un bord extérieur (21), dont au moins une partie est configurée pour sensiblement correspondre à au moins une partie de la périphérie extérieure déduite du plateau tibial et pour reposer sur l'os cortical du tibia.

2. Procédé selon la revendication 1, dans lequel l'utilisation des données d'image électronique comprend la création d'une représentation en deux dimensions, d'une représentation en trois dimensions, ou les deux, de l'articulation de genou comprenant une partie du tibia.

3. Procédé selon la revendication 1, dans lequel le ou les axes de l'articulation de genou sont déterminés en utilisant les données d'image électronique et/ou des données d'image supplémentaires.

4. Procédé selon la revendication 1, dans lequel le ou les axes du genou sont un axe mécanique.

5. Procédé selon la revendication 1, dans lequel le ou les axes du genou sont un axe anatomique.

6. Procédé selon la revendication 1, dans lequel la simulation de la découpe comprend la détermination d'une hauteur de la découpe sur la base d'un point de référence ou d'un point de repère de l'articulation de genou.

7. Procédé selon la revendication 1, comprenant la configuration d'au moins une partie de la périphérie extérieure de l'implant de manière à ce qu'elle repose sur une région d'os spongieux de la surface de découpe simulée du tibia.

8. Procédé selon la revendication 1, comprenant la configuration d'au moins une partie du bord extérieur de la périphérie de l'implant de manière à ce qu'il se trouve au même niveau que la partie correspondante de la périphérie extérieure déduite de la surface de découpe simulée du tibia.

9. Procédé selon la revendication 1, comprenant la configuration d'au moins une partie du bord extérieur de la périphérie de l'implant de manière ce qu'il se trouve en retrait par rapport à la partie correspondante de la périphérie extérieure déduite de la surface de découpe simulée du tibia.
